# FASCICULE DE BREVET EUROPEEN

(11) **EP 0 511 031 B1**
(45) Date de publication et mention de la délivrance du brevet: **15.05.1996**
(21) Numéro de dépôt: 92400721.4
(22) Date de dépôt: 18.03.1992
(51) Int. Cl.: C07D 263/24, C07D 413/12, A61K 31/42

(54) **Dérivés d'aryl-3 oxazolidinone, leur procédé de préparation et leur application en thérapeutique**
3-Aryloxazolidinonderivate, Verfahren zu ihrer Herstellung und ihre Anwendung in der Heilkunde
3-Aryl-oxazolidinon derivatives, process for their preparation and their use in therapeutics

(30) Priorité: 16.04.1991 FR 9104641
(43) Date de publication de la demande: 28.10.1992
(73) Titulaire: SYNTHELABO, F-92350 Le Plessis Robinson (FR)
(72) Inventeur: Jarreau, François-Xavier, F-78000 Versailles (FR); Koenig, Jean-Jacques, F-78600 Maisons Laffitte (FR); Rovei, Vincenzo, F-92500 Rueil Malmaison (FR)
(74) Mandataire: Kedinger, Jean-Paul

(56) Documents cités:
- EP-A- 0 275 742
- EP-A- 0 424 243
- DE-A- 2 923 295
- FR-A- 2 506 769
- GB-A- 2 076 813

## Description

La présente invention a pour objet de nouveaux dérivés d'aryl-3 oxazolidinone-2, leur procédé de préparation et leur application en thérapeutique.

Des dérivés de ce type sont déjà décrits dans EP-A-0 275 742, GB-A-2 076 813, FR-A-2 506 769, DE-A-2 923 295 et EP-A- 0 424 243.

Les dérivés selon l'invention répondent plus précisément à la formule : 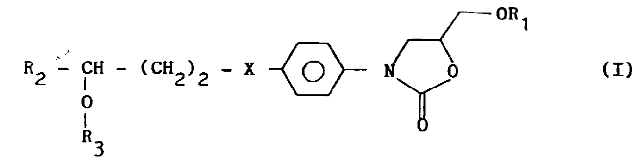 où :
- R₁ représente alkyle en C₁-C₄ ;
- X représente un atome d'oxygène ou un groupe méthylène ;
- R₂ représente alkyle en C₁-C₄ ou CF₃ ;
- R₃ représente un groupe CH₂R₄ dans lequel R₄ est un élément choisi dans le groupe comprenant imidazolyle, pyrazolyle, pyridyle, pyrimidinyle, pyrazinyle, pyridazinyle, quinolyle, isoquinolyle, quinoxalinyle, quinazolinyle et cinnolinyle.

Par ailleurs, il convient de souligner que les dérivés (I) comportent deux atomes de carbone asymétriques. Ils peuvent donc exister sous diverses formes stéréoisomères ou encore sous la forme d'un mélange de ces formes, y compris la forme racémique. La présente invention s'étend par conséquent aux diverses formes ainsi définies.

L'invention s'étend aux N-oxydes des dérivés (I) N-oxydables, ainsi qu'aux sels d'addition d'acide des dérivés (I) et de leur N-oxyde. Ces sels peuvent être formés avec des acides minéraux tels que l'acide chlorhydrique, sulfurique ou phosphorique ou avec des acides organiques tels que l'acide fumarique, maléique, succinique, oxalique, citrique ou tartrique.

Préférence est donnée :
- aux dérivés (I) pour lesquels R₄ = imidazolyle,
- aux dérivés (I) pour lesquels R₄ = pyridyle,
- aux dérivés (I) pour lesquels R₄ = quinolyle,
- aux dérivés (I) pour lesquels R₄ = pyridyle N-oxyde, et
- aux dérivés (I) pour lesquels R₄ = quinolyle N-oxyde.

Parmi les dérivés (I), on citera en particulier :
a) ceux pour lesquels R₁ = alkyle en C₁-C_{4'} X représente oxygène, R₂ = CF₃ et R₄ = pyridyle ;
b) ceux pour lesquels R₁ = alkyle en C₁-C_{4'} X représente oxygène, R₂ = CF₃ et R₄ = pyridyle N-oxyde ;
c) ceux pour lesquels R₁ = alkyle en C₁-C_{4'} X représente oxygène, R₂ = CF₃, R₄ = pyridyle, et dont le carbone asymétrique du cycle oxazolidinone est de configuration (R) ;
d) ceux pour lesquels R₁ = alkyle en C₁-C_{4'} X représente oxygène, R2 = CF3, R4 = pyridyle N-oxyde, et dont le carbone asymétrique du cycle oxazolidinone est de configuration (R) ;
e) ceux pour lesquels R₁ = alkyle en C₁-C₄, X représente oxygène, R2 = CF₃, R4 = pyridyle et dont le carbone asymétrique 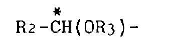 est de configuration (R) ;
f) ceux pour lesquels R₁ = alkyle en C₁-C₄, X représente oxygène, R₄ = pyridyle N-oxyde et dont le carbone asymétrique 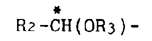 est de configuration (R) ;
g) ceux pour lesquels R₁ = alkyle en C₁-C₄, X représente oxygène, R₂ = CF₃, R₄ = pyridyle et dont le carbone asymétrique 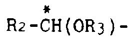 est de configuration (S) ;
h) ceux pour lesquels R₁ = alkyle en C₁-C₄, X représente oxygène, R₄ = pyridyle N-oxyde et dont le carbone asymétrique 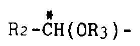 est de configuration (S) ;
i) ceux pour lesquels R₁ =alkyle en C₁-C_{4'} X représente oxygène, R₂ CF3, R₄ = pyridyle et de configuration (R, R) ;
j) ceux pour lesquels R₁ = alkyle en C₁-C_{4'} X représente oxygène, R₂ = CF₃, R₄ = pyridyle N-oxyde et de configuration (R, R) ;
k) les dérivés selon a) à j) où X représente non pas oxygène mais un groupe CH₂ ; et
I) les dérivés selon a) à k) sous la forme de sels d'addition d'acide.

La présente invention a par ailleurs pour objet les procédés de préparation des dérivés (I) et de leur N-oxyde éventuel, ces procédés étant conformes aux schémas 1 à 4 ci-après dans lesquels les symboles R_{1'} X, R₂ et R₃ ont la même signification que dans la formule (I).

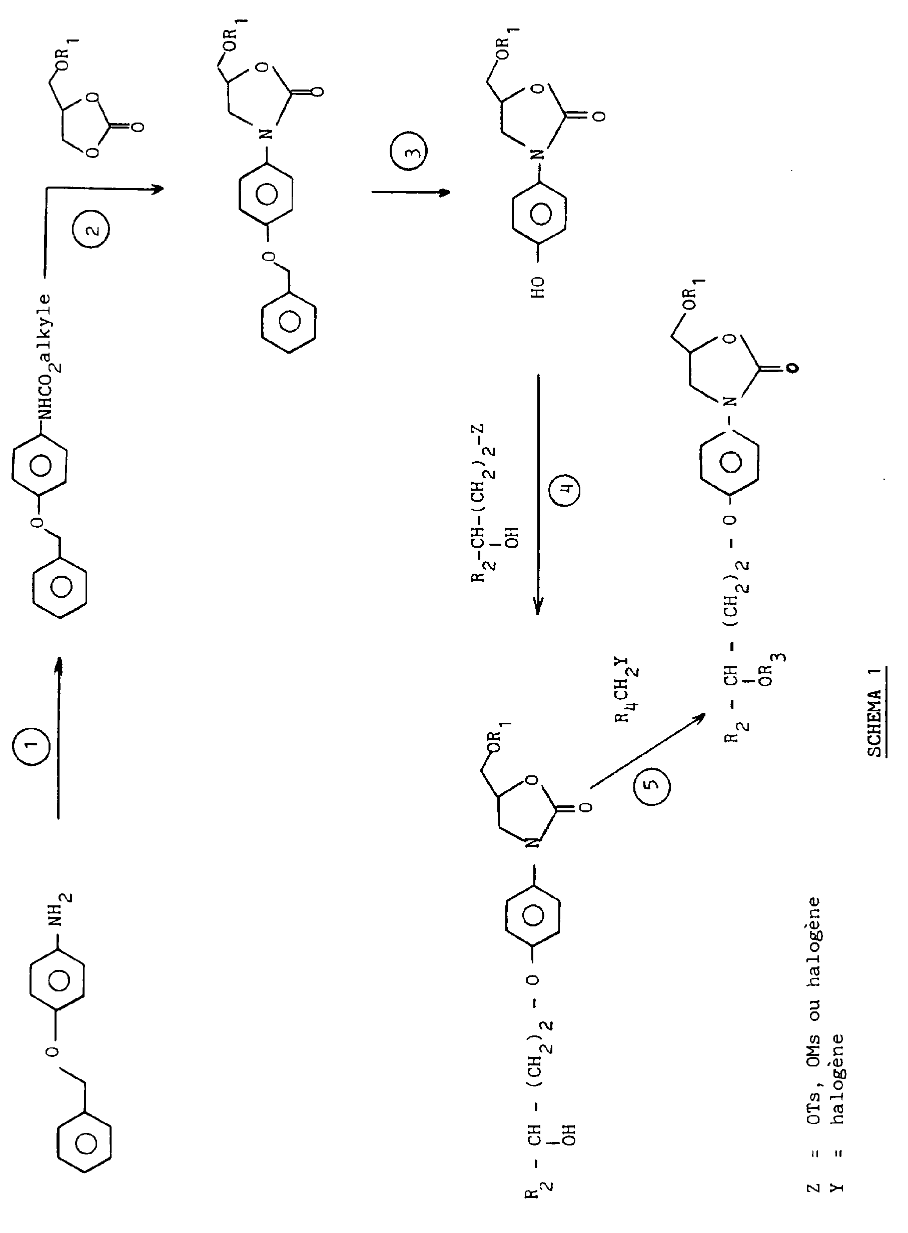

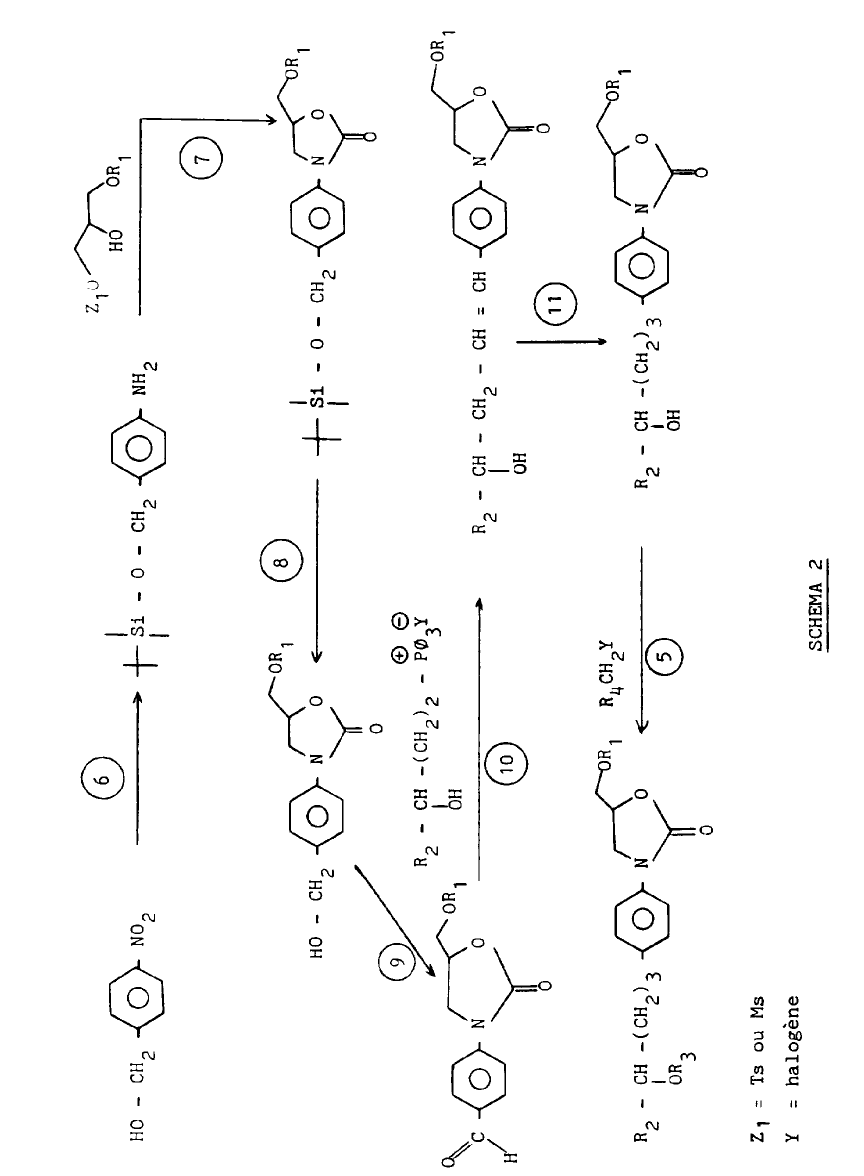

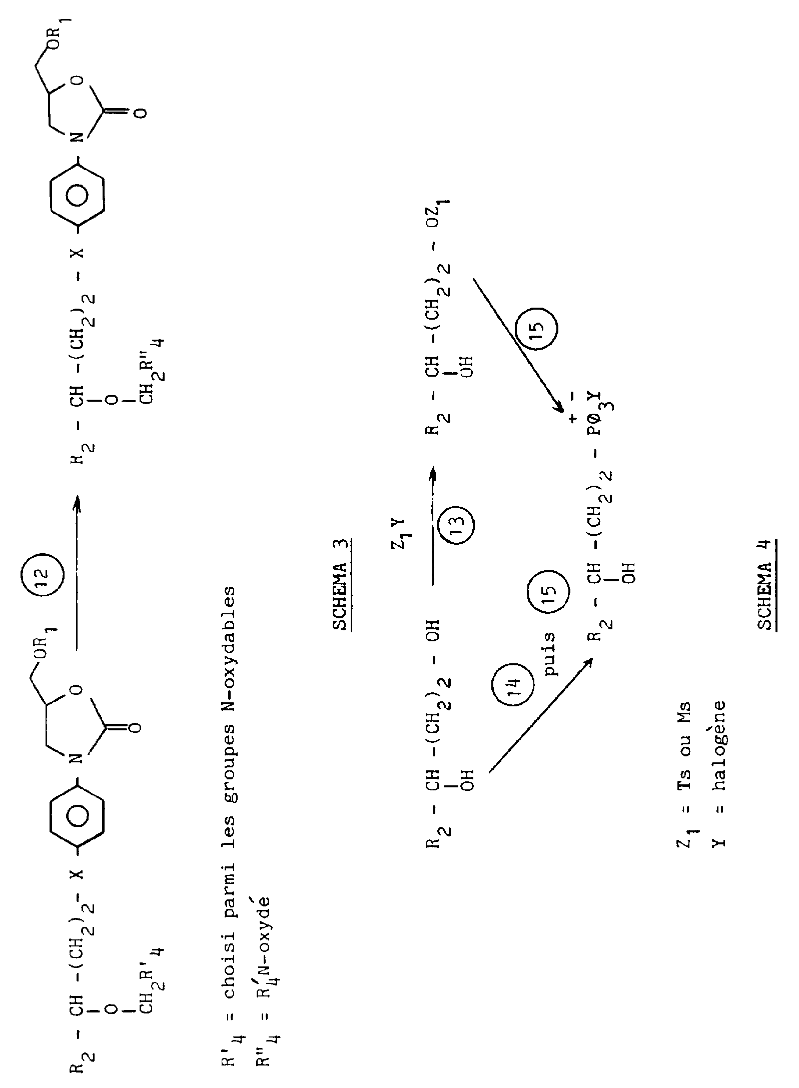

Les indices 1 à 15 apparaissant dans les schémas ci-dessus, ont les significations suivantes :
1 Condensation avec un chloroformiate d'alkyle, comme le chloroformiate d'éthyle, en présence d'une base notamment NaHC0₃ dans un mélange solvant eau-THF, à température ambiante.
2 Condensation à chaud (vers 150° C) en présence d'une base comme K₂CO₃, la réaction conservant la stéréochimie ; ou Condensation dans le toluène en présence de LiBr et de nBu₃PO.
3 Débenzylation dans un solvant alcoolique, comme le méthanol ou l'éthanol en présence d'hydrogène et d'un catalyseur notamment le palladium sur carbone à 10 % humidifié ou non
4 O.alcoylation dans un solvant organique aprotique anhydre comme la méthyléthylcétone ou le DMF et en présence d'une base notamment K₂CO₃, ou
O.alcoylation dans un solvant organique aprotique comme le DMF et/ou le THF et en présence d'un hydrure de métal alcalin comme l'hydrure de sodium.
5 O.alcoylation, notamment dans des conditions de transfert de phase en présence d'un catalyseur comme un bromure d'ammonium quaternaire, par exemple le bromure de tertiobutylammonium, dans un mélange toluène/NaOH aqueux à 50 %, à température ambiante ou à chaud.
6 O.silylation de l'alcool dans un solvant organique aprotique comme le THF en présence d'une base notamment l'imidazole, et de terbutyldiméthylchlorosilane ; puis réduction du dérivé nitré, par la poudre en fer en présence de chlorure d'ammonium
7 Condensation en présence de phosgène et d'une base notamment la diméthylaniline ou diéthylaniline dans un solvant organique comme le chlorure de méthylène ou le dichloroéthane ; puis cyclisation par chauffage dans un solvant organique notamment alcoolique comme l'éthanol en présence d'une base notamment la potasse.
8 Hydrolyse dans un solvant organique notamment le THF en présence d'un composé d'ammonium quaternaire, notamment le fluorure de tétrabutylammonium.
9 Oxydation en présence de chlorure d'oxalyle, de DMSO et d'une base, notamment, la triéthylamine dans un solvant organique aprotique comme le chlorure de méthylène.
0 Condensation en présence d'une base notamment K₂CO₃ et de formamide dans un solvant aprotique notamment le dioxanne, de préférence au reflux,
   ou
Condensation en présence de LDA (lithium diisopropylamide) dans un mélange de solvants notamment DMSO/THF.
0 Hydrogénation sous pression atmosphérique d'hydrogène dans un solvant organique notamment l'acétate d'éthyle en présence d'un catalyseur comme le palladium sur carbone à 10 % humidifié ou non, ou PtO₂, ou
Hydrogénation sous pression d'hydrogène notamment sous 5 atmosphères, en présence de palladium sur carbone à 10 % humidifié ou non, ou Pt02, dans un solvant alcoolique, notamment l'éthanol.
12 N-Oxydation, par exemple par l'acide métachloroperbenzoïque, notamment dans un solvant aprotique comme par exemple le chlorure de méthylène, à température ambiante.
13 Condensation dans un solvant organique notamment la pyridine, THF ou CH₂Cl₂ et d'une base notamment la diméthylamino-4 pyridine ou Et₃N.
14 Conformément à Can. J. Chem. 1968, 46, 86.
15 Conformément à Helv. Chim. Acta 59, 755 (1976).

Il convient d'ajouter que les différentes formes stéréoisomères des composés selon l'invention peuvent être séparées par des méthodes conventionnelles (par exemple méthode de formation d'un sel avec un acide chiral) ou un isomère donné peut être préparé à partir de réactifs chiraux.

Les préparations suivantes sont données à titre d'exemples pour illustrer l'invention :

### Exemple 1 : (benzyloxy-4 phényl)-3 méthoxyméthyl-5 (R) oxazolidinone-2 (MD 200404)

### Stade 1 : Diméthyl-2,2 méthoxyméthyl-4 (S) dioxolane (Numéro de code MD 370486)

A 910 ml d'eau, on ajoute 910 g de NaOH en pastilles puis à température ambiante 5 de chlorure de méthylène, 44,4 g (0,195 mole) de chlorure de benzyltriéthylammonium, 8558,6 g (6,5 moles) de diméthyl-2,2 hydroxyméthyl-4 (S) dioxolane et 1229,5 g (9,75 moles) de sulfate de diméthyle. Le milieu réactionnel est agité 12 h et versé sur l'eau. La phase organique est concentrée. Le composé attendu est obtenu par distillation.
Eb : 45° C sous 10 mm Hg ;
   . [α]²⁰_{D} : + 7.9° (C = 4, CH₃OH) ;
   IR (microcellule) vcm-¹ : 2995, 2940, 2820, 1380, 1370, 840 ;
   RMN¹ (CDCl₃) 8ppm : 1,2 (3H) ; 1,4 (3H) ; 3,35 (3H) ; 3,4-4.4 (3H) ; 4 (2H).

### Stade 2 : Méthoxy-3 propane diol-1,2 (R) (Numéro de code MD 370487)

On chauffe une solution de 950.3 g (6,5 moles) de MD 370486 dans 4510 ml d'eau à 60° C et on ajoute 3,2 ml d'acide chlorhydrique concentré. Puis on ajoute 9 ml de triéthylamine et le milieu réactionnel est concentré et distillé. Le composé attendu est obtenu avec un rendement de 84 %.

Eb = 66° C sous 1 mm Hg ;
. [α]²⁰_{D} : - 6.4° (C = 4, CH₃0H) ;
IR (microcellule) vcm-¹ : 3300, 3500, 2960, 2945, 2910 ;
RMN¹ H(DMSOd6) δppm : 3,2-3,7 (8H) ; 4,5 (2H éch.).

### Stade 3 : Méthoxyméthyl-4 dioxolane-1,3 one-2 (S) (Numéro de code MD 360287)

On chauffe un mélange de 14 g (0,132 mole) de méthoxy-3 propane diol-1,2 (R) et de 31,16 g (0,264 mole) de carbonate de diéthyle en présence de 0,108 g d'hydrure de sodium à 50 % jusqu'à distillation de l'alcool formé. Lorsque la réaction est complète, le produit attendu est distillé.

Eb 0,3 = 117° C ;
Rendement : 93 % ;
. [α] 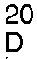 -32,2° (C = 1, CH₂Cl₂) ;
IR (microcellule)vC0 : 1790 cm⁻¹ ;
RMN¹ H (CDCl₃) δppm : 3,4 (3H) ; 3,6 (2H) ; 4,3-4,9 (3H).

### Stade 4 : N-éthoxycarbonyl benzyloxy-4 aniline (Numéro de code MD 360343)

A une solution de 10 g (10-³ mole) de benzyloxy-4 aniline dans 90 ml de THF et 10 ml d'eau, on ajoute 6,3 g de bicarbonate de sodium, puis 5,28 ml (55.10-³ mole) de chloroformiate d'éthyle. Après 18 heures d'agitation, le milieu réactionnel est filtré et concentré. Le résidu est repris dans l'acétate d'éthyle. La solution organique est lavée à l'eau, séchée sur Na₂S0₄ et concentrée. Le produit est obtenu avec un rendement de 91 %.
F=98°C:
IR (KBr)vcm-¹ : 3320, 1700, 1510-1530, 1230 ;
RMN¹ H (CDCl₃) δppm : 1,2 (3H) ; 4,2 (2H) ; 5 (2H) ; 6,7 (1 H); 6,9 (2H) ; 7,2 (2H).

### Stade 5 : (Benzyloxy-4 phényl)-3 méthoxyméthyl-5 (R) oxazolidinone-2 (Numéro de code MD 200404)

On chauffe à 160° C sous agitation 1 g (3,6.10-³ mole) de MD 360343, 0,099 g (0,72.10-³ mole) de K₂CO₃ et 0,586 g (4,5 10-³ mole) de MD 360287 (obtenu au stade 3) pendant 3 heures. Après refroidissement, le milieu réactionnel est repris dans le chlorure de méthylène, lavé à l'eau, séché sur Na₂SO₄ et concentré. Le produit est recristallisé dans l'isopropanol.

Rendement = 71 % :
F=101°C;
. [α]²⁰_{D} : - 41,5° (C = 1, CH₂Cl₂).

Par un mode opératoire identique mais à partir des réactifs appropriés, on a obtenu :
la (benzyloxy-4 phényl)-3 méthoxyméthyl-5 (S) oxazolidinone-2 (Numéro de code MD 340190)
. F=101°C;
. [α]²⁰_{D} : + 41.9° (C = 1, CH₂Cl₂) ; ainsi que :
- la (benzyloxy-4 phényl)-3 éthoxyméthyl-5 (R) oxazolidinone-2 (Numéro de code MD 230 242)
. F = 78° C;
. [α]²⁰_{D} : -35.9° (C = 1 CH₂Cl₂) ;
IR (KBr)vcm-¹ : 1750, 1735.

### Exemple 2 : (Hydroxy-4 phényl)-3 méthoxyméthyl-5 (R) oxazolidinone-2 (Numéro de code MD 200405)

A une solution de 13 g (0,047 mole) de composé MD 200404 dans 80 ml d'éthanol et 40 ml de CH₂Cl₂ en présence de 2,6 g de Pd/C à 10 % humidifié à 50 %, on fait passer un courant d'hydrogène sous pression normale.

Lorsque la réaction est complète, la solution est filtrée et concentrée. Le produit attendu est obtenu avec un rendement de 100 %.
F=112°C;
. [α]²⁰_{D} : - 67° (C = 1, CH₃OH) ;
IR (KBr) vcm⁻¹ : 3260, 1730.

Par un procédé identique mais à partir des réactifs correspondants, on obtient :
- la (hydroxy-4 phényl)-3 méthoxyméthyl-5 (S) oxazolidinone-2 (Numéro de code MD 200717)
   . F=114°C;
   . [α]²⁰_{D} : + 66° (C = 1, CH₃0H) ;

   ainsi que :
- la (hydroxy-4 phényl)-3 éthoxyméthyl-5 (R) oxazolidinone-2 (Numéro de code MD 230 243)
   . F=92°C;
   . [α]²⁰_{D} : - 58,9° (C = 1, CH₃OH) ;
   . IR (KBr) vcm⁻¹ : 3300, 1770.

### Exemple 3 : [(trifluoro-4;4;4 hydroxy-3 (R) butoxy)-4 phényl]-3 méthoxyméthyl-5 (R) oxazolidinone-2 (Numéro de code MD 370503)

### Stade 1 : Trifluoro-4,4,4 tosyloxy-1 butanol-3 (R) (Numéro de code MD 230099)

A une solution de 120 g (0,833 mole) de trifluoro-4,4,4 butanediol-1,3 (R) (C.A. 111, 31747p) dans 335 ml de pyridine, on ajoute 0,12 g de diméthylamino-4 pyridine et une solution de 198,4 g (1,041 mole) de chlorure de tosyle dans 200 ml de CH₂Cl₂. Après 1 heure 20 mn d'agitation, on ajoute 1,2 | de CH₂Cl₂ et 1,5 | d'eau. La phase organique est concentrée et le produit est purifié par chromatographie [silice ; éluant : Heptane (80) / acétate d'éthyle (20)] et est obtenu avec un rendement de 78 %. II est utilisé directement pour l'étape suivante.

### Stade 2 : [(trifluoro-4,4,4 hydroxy-3 (R) butoxy)-4 phényl]-3 méthoxyméthyl-5 (R) oxazolidinone-2 (Numéro de code MD 370503)

A une solution de 192,1 g (0,644 mole) du composé MD 200405 (exemple 2) dans 400 ml de DMF on ajoute 161,5 g de K₂C0₃ et chauffe à 90° C, puis la suspension du composé MD 230099 dans 200 ml de DMF. Après 1 heure, on refroidit le milieu réactionnel et ajoute 1,2 1 de toluène par litre d'eau.

Après extraction de la phase aqueuse au toluène, les phases organiques sont concentrées à sec. Le produit est recristallisé dans un mélange éthanol-éther isopropylique.
Rendement = 60,8 % ;
F=101°C;
RMN¹ H (CDCl₃) 8 ppm : 2,05 (2H) ; 3,4 (3H) ; 3,6 (2H) ; 3,6-4,4 (6H dont 1 échangeable) ; 4,6 (1 H) ; 6,8 (2H) ; 7;3
(2H).
RMN¹³C (DMSOd6) : Cq : 154,6; 154,4; 125;9 (¹JCF:289;6 Hz); 131,8 ; CH : 119,8 ; 114,8 ; 71,3; 65,4 (²JCF : 30,4 Hz) ; CH₂ : 72,5 ; 63,2 ; 46,6 ; 29,4 ; CH₃ : 58,7 ;
IR (KBr) vcm-¹ : 3400; 1730, 1720 ;
D [α]²⁰_{D} : - 11;5° (C = 1, CH₂Cl₂).

De la même manière mais à partir du trifluoro-4,4;4 tosyloxy-1 butanol-3 (S) et du composé MD 200405 a été obtenu la [(trifluoro-4,4,4 hydroxy-3 (S) butoxy)-4 phényl]-3 méthoxyméthyl-5 (R) oxazolidinone-2 (Numéro de code MD 370504) :
F=121°C;
. [α]²⁰_{D} : - 59,7° (C = 1, CH2Cl₂) ;
RMN¹ H (CDCl₃) δppm : 2,1 (2H) ; 3,4 (4H dont 1 éch.) ; 3;6 (2H) ; 3,7-4,4 (5H) ; 4,7 (1H) ; 6,8 (2H) ; 7,4 (2H) ; IR (KBr) vcm-1 : 3420, 1735.

A partir du composé racémique trifluoro-4,4,4 tosyloxy-1 butanol-3 (Numéro de code MD 370272) et du composé MD 200405 a été obtenu dans les mêmes conditions le mélange de diastéréoisomères [(trifluoro-4,4,4 hydroxy-3 bu- toxy)-4 phényl]-3 méthoxyméthyl-5 (R) oxazolidinone-2 (Numéro de code 230016) :
RMN¹H (CDCl₃ + DMSO) δppm : 1,8-2,3 (2H) ; 3,4 (3H) ;
3,6 (2H) ; 3,8-4,4 (5H) ; 4,7 (1 H) ; 5,3 (1 H éch.) ;
6,9 (2H) ; 7.4 (2H) ;
IR (KBr) vcm-¹ : 3400, 1755, 1735 ;
F=103°C;
. [α]²⁰_{D} : - 35,2° (C = 1, CH₂Cl₂).

De la même manière mais à partir des matières correspondantes ont été obtenus :
- [(Trifluoro-4.4;4hydroxy-3(S)butoxy)-4phényl]-3méthoxyméthyl-5(S)oxazolidinone-2(Numérodecode230154)
   . [α]²⁰_{D} : + 9,9° (C = 1, CH₂Cl₂) ;
   . F=100°C;
- [(Trifluoro-4,4;4 hydroxy-3 (R) butoxy)-4 phényl]-3 méthoxyméthyl-5 (S) oxazolidinone-2 (Numérode code 230151 )
   . [α]²⁰_{D} : + 59,2° (C =1, CH₂Cl₂) ;
   . F =123° C :
- [(Trifluoro-4,4;4 hydroxy-3 (R) butoxy)-4 phényl]-3 éthoxy méthyl-5 (R) oxazolidinone-2 (Numéro de code 230197)
   . F=91°C;
   . [α]²⁰_{D} : -11,4° (C = 1, CH₃0H) ;
   . IR (KBr) vcm¹ : 3400, 1750, 1735 ;
   . RMN¹H (CDCl₃) δppm : 1,1 (3H) ; 3,3-4,4 (9H) ; 4,7(1H) ; 6,3(1Héch.) ; 6,9(2H) ; 7,4(2H);
- [(Hydroxy-3 (R) butoxy)-4 phényl]-3 méthoxyméthyl-5 (R) oxazolidinone-2 (Numéro de code MD 370120)
   . F=76° C;
   . [α]²⁰_{D} : - 50,7° (C =1, CH₂Cl₂) ;
- [(Hydroxy-3 (R) butoxy)-4 phényl]-3méthoxyméthyl-5 (S) oxazolidinone-2 (Numéro de code MD 370123)
   . F=44° C
   . [α]²⁰_{D}: + 33° (C = 1, CH₂Cl₂) ;
- [(Hydroxy-3 (S) butoxy)-4 phényl]-3 méthoxyméthyl-5 (S) oxazolidinone-2 (Numéro de code MD 370121)
   . F = 76° C;
   . [α]²⁰_{D} : + 50,4° (C =1, CH₂₀l₂)
- [(Hydroxy-3 (S) butoxy)-4 phényl]-3 méthoxyméthyl-5 (R) oxazolidinone-2 (Numéro de code MD 370122)
   . [α]²⁰_{D} = - 33,4° (C = 1, CH₂Cl₂) ;
   . RMN¹ H (CDCl₃) δppm : 1,2 (3H) ; 1;8 (2H) ; 2,2 (1 H éch.) ; 3,4 (3H) ; 3,6 (2H) ; 3,7-4,2 (5H) ; 4,7 (1 H) ; 6;8 (2H) ; 7,4 (2H) ;
   RMN¹³C (CDCl₃) δppm : Cq : 155,6 ; 154,9; 131,6; CH : 120,2 ; 115 ; 71,3; 65.6 ; CH₂ : 72,7 ; 65.9 : 47,6 ; 38,2 ; CH3 : 59,6 ; 23.6 ;
   IR (KBr) vcm-¹ : 3380-3400, 1730, 1755 ;
   . F = 49° C;
- [(Hydroxy-3 butoxy)-4 phényl]-3 méthoxyméthyl-5 (R) oxazolidinone-2 (Numéro de code MD 370284)
   . RMN¹ H (CDCl₃) δppm : 1,2 (3H) ; 1;8 (2H) ; 2,6 (1 H éch.) ; 3,4 (3H) ; 3,6 (2H) ; 3,7-4,2 (5H) ; 4,7 (1 H) ; 6,8 (2H) ; 7,4 (2H) ;
   . IR (KBr) vcm-¹ : 3400, 1745, 1730 ;
   . [α]²⁰_{D} = - 41,5° (C = 1, CH₂Cl₂).

### Exemple 4 : Mélange racémique de diastéréoisomères d'[(hydroxy-3 butoxy)-4 phényl]-3 méthoxyméthyl-5 oxazolidinone-2 (Numéro de code MD 370047)

A une solution de 27,5 g (0,112 mole) de tosyloxy-1 butanol-3 dans 250 ml de méthyléthylcétone, on ajoute 28,2 g (0,2 mole) de K₂CO₃ et 22,8 g (0,102 mole) d'(hydroxy-4 phényl)-3 méthoxyméthyl-5 oxazolidinone-2 (numéro de code 780232). On chauffe au reflux pendant 4 h 30. Après filtration et concentration, le résidu est repris dans 200 ml de CH₂Cl₂, la phase organique est lavée à l'eau saturée de NaCI, séchée sur Na₂S0₄ et concentrée. Après purification par flash chromatographique [silice, éluant : CH₂Cl₂ (98) / CH₃0H (2)], on obtient le produit attendu avec un rendement de 70 %.
F = 58° C
RMN¹ H (CDCl₃) δppm : 1,2 (3H) ; 1,8 (2H) ; 2,5 (1 H éch.) ; 3,4 (3H) ; 3,6 (2H) ; 3,7-4,2 (5H) ; 4,7 (1H) ; 6,8 (2H) ;
7,4 (2H) ;
IR (KBr) vcm-¹ : 3400, 1750, 1730.

De la même manière, mais à partir de trifluoro-4,4,4 tosyloxy-1 butanol-3 et de l'(hydroxy-4 phényl)-3 méthoxyméthyl-5 oxazolidinone-2 (numéro de code MD 780232), on a obtenu le mélange des diastéréoisomères racémique [(trifluoro-4,4,4 hydroxy-3 butoxy)-4 phényl]-3 méthoxyméthyl-5 oxazolidinone-2 (numéro de code MD 370167):
F=89°C:
RMN¹ H (CDCl₃) ; δppm : 2,05 (2H) ; 3,4 (3H) ; 3,5 (1 H, éch.) ; 3,6 (2H) ; 3,7-4,3 (5H) ; 4;7 (1H) ; 6,8 (2H), 7,3 (2H) ;
IR (KBr) vcm-¹ : 3400, 1750, 1785.

### Exemple 5 : [(Hydroxy-4 (R) pentyl)-4 phényl]-3 méthoxyméthyl-5 (R) oxazolidinone-2 (Numéro de code MD 230238)

### Stade 1 : Terbutyl diméthyl silyloxy méthyl-4 nitro-1 benzène (Numéro de code MD 230245)

A une solution de 465,4 g (3,039 moles) de paranitrobenzylalcool dans 2,5 1 de DMF, on ajoute 310 g (4,559 moles) d'imidazole puis 504 g (3,347 moles) de terbutyl diméthylchlorosilane. Après 1 h d'agitation à température ambiante, le milieu réactionnel est versé sur l'eau. La phase aqueuse est extraite par du chlorure de méthylène. La phase organique est séchée sur Na2S04 et concentrée. On obtient une huile qui correspond au composé attendu :
RMN¹ H (CDCl₃) δ ppm : 0,2 (6H) ; 1 (9H) ; 4,9 (2H) ; 7,6 (2H) ; 8,2 (2H) ;
IR (microcellule) vcm-¹ : 1520, 1340; 1030, 840.

### Stade 2 : Terbutyl diméthylsilyloxy méthyl-4 aniline (Numéro de code MD 230246)

A 772 ml de chlorure d'ammonium 0,1 N, on ajoute 77,2 g (0,288 mole) du composé précédent MD 230245 et 120,9 g de poudre de fer et on chauffe à reflux 2 h. Après refroidissement, on ajoute 20 ml d'ammoniaque concentrée, le milieu réactionnel est filtré et extrait par le toluène. La phase organique est lavée à l'eau, séchée au Na2S04 et concentrée pour obtenir le composé attendu :
Eb 0,01 mm Hg : 88-93° C ;
RMN¹ H (CDCl₃) 8 ppm : 0,2 (6H) ; 1,05 (9H) ; 3,6 (2H) ; 4,8 (2H) ; 6,75 (2H) ; 7,2 (2H) ;
IR (microcellule) vcm-¹ : 3450, 3350.

### Stade 3 : Tosylate de méthoxy-3 propane-diol-1,2 (S) (Numéro de code MD 370488)

Une solution de 371,4 g (3,5 moles) de MD 370487) dans 100 ml de toluène est refroidie à 13° C et on ajoute 565 ml de pyridine puis petit à petit une solution de 700,6 g (3,675 moles) de chlorure de paratoluène sulfonyle dans 775 ml de toluène.

Le milieu réactionnel est agité pendant 12 h et versé sur l'eau. La phase organique est lavée à l'acide chlorhydrique 2N et concentrée. Le produit attendu est obtenu avec un rendement de 58 % après chromatographie [silice, éluant: CH₂Cl₂ (50) / éther de pétrole (50)] ;
. [α]²⁰_{D} : + 5.3° (C = 4, CH₃OH) ;
IR (microcellule) vcm-¹ : 3500, 1335, 1185. 1170 ;
RMN¹ H (CDCl₃) δ ppm : 2,4 (3H) ; 3,1 (1 H éch.) ; 3,2-3,6(5H); 3,8-4,2 (3H).

### Stade 4 : [(Terbutyl diméthyl silyloxy méthyl)-4 phényl]-3 méthoxyméthyl-5 (R) oxazolidinone-2 (Numéro de code MD 230247)

A une solution de 43,8 g (0,168 mole) de MD 370488 dans 200 ml de toluène, on ajoute 130 ml d'une solution toluénique de phosgène 1,93 molaire puis, goutte à goutte, 37,8 g (0;252 mole) de diéthylaniline. Après refroidissement, on ajoute de l'eau glacée et la phase organique est décantée et séchée sur Na2S04. Cette solution est alors additionnée à une solution de 40 g (0,168 mole) de MD 230246 et de 20,5 g (0,168 mole) de diméthylaminopyridine dans 600 ml de toluène. Après ½ heure d'agitation, le milieu réactionnel est versé sur l'eau et la phase organique est lavée avec une solution de bicarbonate de sodium puis par une solution saturée en NaCI. Après concentration, le produit obtenu (84;5 g) est mis en solution dans 800 ml d'éthanol auquel est ajouté 12,2 g (0,218 mole) de KOH en pastilles. Après ½ heure d'agitation, le milieu réactionnel est versé sur l'eau et extrait au chlorure de méthylène. La phase organique est séchée sur Na2S04 et concentrée. Le produit attendu est obtenu après chromatographie [silice, éluant : acétate d'éthyle (30) / heptane (70)] avec un rendement de 63 % :
. [α] 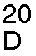 : - 46.2° (C = 1, CH₃OH) ;
IR (KBr) vcm-¹ : 1755, 1735 ; . RMN¹H (CDCl₃) δ ppm : 0 (6H) ; 1 (9H) ; 3,4 (3H) _{;} 3,6 (2H) ;
. 3,8-4,2 (2H) ; 4,7 (3H) ; 7.5 (4H) ;

F < 50° C.

### Stade 5 : [Hydroxyméthyl-4 phényl]-3 méthoxyméthyl-5 (R) oxazolidinone-2 (Numéro de code MD 230248)

Une solution de 29,2 g (0,083 mole) de MD 230247 et 7,8 g (0,025 mole) de fluorure de tétrabutylammonium trihydraté dans 200 ml de THF est agitée 12 h à température ordinaire et le milieu réactionnel est concentré. Le produit attendu est obtenu après chromatographie [silice, éluant : acétate d'éthyle (50) / heptane (50)]:
F=65°C:
IR (KBr) vcm-¹ : 3400, 1750, 1720 ;
RMN¹ H (CDCl₃) δppm : 2,4 (1 H éch.) ; 3,35 (3H) ; 3,6 (2H) ; 3,8-4,2 (2H) ; 4,6 (2H) ; 7,35 (4H).

### Stade 6 : (carboxaldéhydo-4 phényl)-3 méthoxyméthyl-5 (R) oxazolidinone-2 (Numéro de code MD 230256)

A une solution refroidie à -60° C de 12,46 g (0,0982 mole) de chlorure d'oxalyle dans 80 ml de chlorure de méthylène, on introduit en 20 mn une solution de 12,76 g (0,1630 mole) de DMSO dans 80 ml de chlorure de méthylène. 40 mn plus tard, on ajoute une solution de 19,6 g (0,0818 mole) de MD 230248 dans 80 ml de chlorure de méthylène puis 1,4 g (0,409 mole) de triéthylamine. Après retour à température ambiante, on ajoute 300 ml d'eau. La phase organique est lavée à l'eau, séchée et concentrée. Le produit attendu a été obtenu après purification par chromatographie [silice, éluant : acétate d'éthyle (70) / heptane (30)] avec un rendement de 80 %.
F=96°C:
. [α]²⁰_{D} : - 73,4° (C = 1, CH₂Cl₂) ;
IR (KBr) vcm-¹ : 1740, 1690;
RMN¹ H (CDCl₃) 8ppm : 3,4 (3H) ; 3,7 (2H) ; 3,8-4,3 (2H) ; 4_{;}8 (1 H) ; 7,8 (4H) ; 9,8 (1 H).

### Stade 7 : [(Hydroxy-4 (R) pentyl)-4 phényl]-3 méthoxyméthyl-5 (R) oxazolidinone-2 (Numéro de code MD 230238)

Une solution de 3,3 g (0,00712 mole) d'iodure d'hydroxy-2 (R) butyl triphénylphosphonium (Helv. Chim. Acta, 59, 755-757, 1976)_{;} de 1,34 g (0,00569 mole) de MD 230256 et 2,9 g (0,0213 mole) de K₂CO₃ dans 10 ml de dioxanne et 1,5 ml de formamide est chauffée au reflux 20 h. Après filtration et concentration, le produit insaturé obtenu est purifié en le dissolvant dans 30 ml de DMF et on ajoute 0,58 g d'imidazole et 0,94 g (0,00625 mole) de terbutyl diméthylchlorosilane. Après 24 h d'agitation, le milieu réactionnel est versé sur l'eau. Le produit silylé est extrait au chlorure de méthylène et purifié par chromatographie [silice, éluant : acétate d'éthyle (50) / heptane (50)] avec un rendement de 36 %. 0,84 g du produit résultant est mis en solution dans 15 ml de THF en présence de 0,65 g de fluorure de tétrabutylammonium pendant 12 h. Après concentration et purification par chromatographie [silice, éluant : acétate d'éthyle (70) / heptane (30)], 0,53 g (0,0018 mole) du produit insaturé purifié en solution dans 10 ml de méthanol en présence de palladium sur carbone à 10 % (humide à 50 %) est hydrogéné sous pression normale. Le produit attendu est obtenu avec un rendement de 55 % après chromatographie [silice, éluant : acétate d'éthyle (60) / heptane (40)].
. [α] 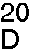 : - 45,8° (C = 1, CH₂Cl₂) ;
IR (KBr) vcm-¹ : 3400, 1735;
F=47°C:
RMN¹ H (CDCl₃) δ ppm : 1,2 (3H) ; 1,5 (4H) ; 1,8 (1 H éch.) ; 2,6 (2H) ; 3,4 (3H) ; 3,6 (2H) ; 3,7-4,2 (3H) ; 4,7 (1 H) ;
7,2 (2H) ; 7,4 (2H).

De la même façon ont été obtenues :
- [(hydroxy-4 (S) pentyl)-4 phényl]-3 méthoxyméthyl-5 (R) oxazolidinone-2 (Numéro de code MD 230239)
   . F = 53° C;
   . [α]²⁰_{D} : - 35,9° (C = 1, CH₂Cl₂) ;
   IR (KBr) vcm-¹ : 3400, 1740 ;
   . RMN¹ H (CDCl₃) δ ppm : 1,1 (3H) ; 1,6 (5H dont 1 éch.) ; 3,4 (3H) ; 3;6 (2H) ; 3,7-4,2 (3H) ; 4,7 (1 H) ; 7,1 (2H) ; 7;4 (2H)
- [(hydroxy-4 pentyl)-4 phényl]-3 méthoxyméthyl-5 (R) oxazolidinone-2 (Numéro de code 230082)
   . [α]²⁰_{D} : - 56,3° (C = 1, MeOH) ;
   . RMN¹ H (CDCl₃) δ ppm : 1,15 (3H) 1,55 (5H) ; 2,6 (2H) ; 3,4 (3H) ; 3,6 (3H) ; 3,9 (2H) 4,65 (1H) ; 7,1 (2H) ; 7;4 (2H) ;
   IR (microcellule) vcm-¹ : 3500-3400, 1750.

### Exemple 6 : [(trifluoro-5,5,5 hydroxy-4 pentyl)-4 phényl]-3 méthoxyméthyl-5 (R) oxazolidinone-2 (Numéro de code MD 360207)

Obtenue selon le mode opératoire de l'exemple 5, stade 7, à partir des matières premières correspondantes :
Huile ;
IR (microcellule) vcm-¹ : 3410, 1735 ;
RMN¹ H (CDCl₃) δ ppm : 1 (4H) ; 2,7 (2H) ; 3,4 (3H) ; 3,6 (3H dont 1 éch.) ; 4 (1 H) ; 4,7 (1 H) ; 7,1 (2H) ; 7,4 (2H).

### Exemple 7 : Trifluoro-4,4,4 iodo-1 butanol-3 (Numéro de code MD 360253)

A une solution de 2,04 g (0,0068 mole) de MD 370272 (trifluoro-4,4,4 tosyloxy-1 butanol-3) dans 20 ml d'acétone, on ajoute 2,56 g (0,0171 mole) de K| et chauffe à reflux une nuit. Après filtration et concentration, le produit est obtenu par chromatographie [silice, éluant : heptane (80) / acétate d'éthyle (20)].
IR (microcellule) vcrm⁻¹ : 3400 ;
RMN¹ H (CDCl₃) δ ppm : 1,9 - 2,5 (2H) ; 2,4 (1 H éch.) ; 3;35 (2H) ; 4;2 (1 H).

### Exemple 8 : lodure de trifluoro-4;4,4 hydroxy-3 butyl triphénylphosphonium (Numéro de code MD 360254)

On chauffe une nuit 35,6 g (0,14 mole) de MD 360253 et 36,8 g (0;14 mole) de triphénylphosphine dans le dioxane à reflux. Le produit est filtré et séché.
Rendement : 72 % ;
F = 159° C.

### Exemple 9 : [[Trifluoro-4,4,4 (pyridyl-3 méthoxy)-3 (R) butoxy]-4 phényl]-3 méthoxyméthyl-5 (R) oxazolidinone-2 (Numéro de code MD 360411 )

A 100 ml de toluène on ajoute 9,6 g (0,03 mole) du composé MD 370503, 9,8 g (0,06 mole) de chlorure de picolyl-3, chlorhydrate, 0,97 g (0,003 mole) de bromure de tétrabutylammonium et 14,4 g de NaOH à 50 % dans l'eau.

Après une nuit d'agitation; le milieu réactionnel est versé sur l'eau. Le mélange est acidifié par l'acide chlorhydrique. La phase aqueuse est lavée au toluène, puis alcalinisée par une solution de carbonate de sodium et extraite par l'acétate d'éthyle. La phase organique est lavée à l'eau, séchée sur sulfate de sodium et concentrée. Le produit attendu est obtenu après chromatographie sur colonne [Si0₂, éluant : CH₂Cl₂ (97) / CH₃0H (3)] avec un rendement de 45 % (huile).
IR (microcellule) vcm-¹ : 1747 ;
. [α]²⁰_{D} : + 54,9° (C = 1, CH₂Cl₂) ;
RMN¹ H (CDCl₃) δ ppm : 1,8-2,3 (2H) ; 3,4 (3H) ; 3,6 (2H) ; 3,8-4,3 (5H) ; 4,4-5 (3H) ; 6,8 (2H) ; 7-7,6 (4H) ; 8,5 (2H). Analyse élémentaire pour C₂₁H₂₃F₃N₂O₅ :

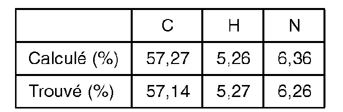

### Oxalate du composé MD 360411

A une solution de 6,190 g (0,014 mole) du composé base MD 360411 dans 20 ml d'acétate d'éthyle, on ajoute une solution de 1,265 g (0,014 mole) d'acide oxalique dans 30 ml d'acétate d'éthyle. Le précipité obtenu est filtré et recristallisé dans l'acétate d'éthyle.
Rendement = 69 % ;
F=80°C:
. [α]²⁰_{D} : + 58,8° (C = 1, CH2C12) ;

Analyse élémentaire pour C₂₃H₂₅F₃N₂O₉ + 0;49 % H₂O :

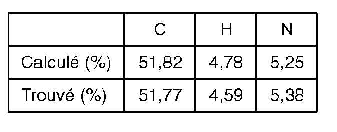

De la même manière, mais à partir des matières correspondantes ont été obtenues :
- [[trifluoro-4,4;4 (pyridyl-4 méthoxy)-3 (R) butoxy]-4 phényl]-3 méthoxyméthyl-5 (R) oxazolidinone-2 (Numéro de code MD 290057) :
. F=91,5° C;
. IR (KBr) vcm⁻¹ : 1735 ;
. [α]²⁰_{D} : + 51,6° (C = 1, CH₂Cl₂) ;
. RMN¹H (CDCl₃) δppm : 1,8-2;4 (2H) ; 3,4 (3H) ; 3,6 (2H) ; 3,7-4,3 (5H) ; 4,4-5 (3H) ; 6,8 (2H) ; 7,2 (2H) ; 7;4 (2H) ; 8,5 (2H) ;

Analyse élémentaire pour C₂₁H₂₃F₃N₂O₅:

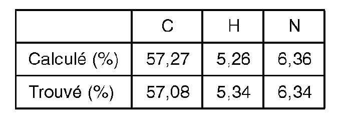

- [[trifluoro-4,4;4 (pyridyl-2 méthoxy)-3 (R) butoxy]-4 phényl]-3 méthoxyméthyl-5 (R) oxazolidinone-2 (Numéro de code MD 290104)
Liquide ;
IR (microcellule) vcm-¹ : 1750 ;
. [α]²⁰_{D} : + 44,7° (C = 1, CH₂Cl₂) ;
. RMN¹ H (CDCl₃) δppm : 1,8-2,4 (2H) ; 3,4 (3H) ; 3,6 (2H) ; 3,75-4,4 (5H) ; 4,6-5,1 (3H) ; 6,8 (2H) ; 7,1 (1 H) ; 7;35 (1 H) ; 7,4 (2H) ; 7,6 (1 H) ; 8,45 (1 H) ; Analyse élémentaire pour C₂₁H₂₃F₃N₂O₅

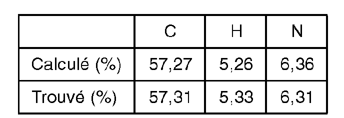

Par mise en oeuvre des protocoles opératoires de cet exemple 9, on obtient d'autres dérivés (I) et sels, à partir des composés obtenus aux exemples 3, 4 et 5.

### Exemple 10 : [[trifluoro-4,4,4 (N-oxyde-pyridyl-3 méthoxy)-3 (R) butoxy]-4 phényl]-3 méthoxyméthyl-5 (R) oxazolidinone-2 (Numéro de code MD 290065)

A une suspension de 3,4 g (0,011 mole) d'acide métachloroperbenzoïque à 55 % dans 40 ml de CH₂Cl₂, on ajoute à 10° C une solution de 4 g (0,0091 mole) du composé MD 360411 dans 20 ml de chlorure de méthylène. Puis on laisse en agitation 24 heures. Le milieu réactionnel est alcalinisé par l'ammoniaque 3N. La phase aqueuse est extraite par CH₂Cl₂ et les phases organiques sont lavées à l'eau, séchées et concentrées. Le produit attendu est obtenu après chromatographie sur colonne [Si0₂, éluant : CH₂Cl₂ (97) / méthanol (3)]:
Rendement : 84 % ;
. [α]²⁰_{D} : + 67,1 ° (C = 1, CH₂Cl₂) ;
IR (KBr) vcm⁻¹: 1745:
RMN¹ H (CDCl₃) δppm : 1,8-2;3 (2H) ; 3,4 (3H) ; 3,6 (2H) ; 3,7-4,3 (5H) ; 4,35-4,8 (3H) ; 6,8 (2H) ; 7-7,5 (4H) ; 8 (2H) ;

Analyse élémentaire pour C₂₁H₂₃F₃N₂O₆ + 0;76 % H₂O :

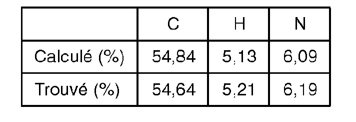

De la même manière ont été obtenues à partir des matières premières correspondantes :
[[Irifluoro-4,4,4 (N-oxyde pyridyl-4 méthoxy)-3 (R) butoxy]-4 phényl]-3 méthoxyméthyl-5 (R) oxazolidinone-2
[[Irifluoro-4,4,4 (N-oxyde-pyridyl-2 méthoxy)-3 (R) butoxy]-4 phényl]-3 méthoxyméthyl-5 (R) oxazolidinone-2

### Exemple 11 : Trifluoro-4,4,4 tosyloxy-1 butanol-3 (racémique) et trifluoro-4,4,4 tosyloxy-1 butanol-3 (S)

Obtenus par mise en oeuvre du protocole opératoire du stade 1 de l'exemple 3 à partir des diols (respectivement racémique et S) eux-mêmes préparés selon C.A. 111, 31747p à partir des acides correspondants décrits dans Chimia, vol. 44, No. 4, pages 90-92, 1990.

Les dérivés de formule (1), leurs N-oxyde et sels pharmaceutiquement acceptables ont été étudiés chez l'animal de laboratoire et ont montré des activités pharmacologiques notamment dans le domaine psychotrope, en particulier comme anxiolytiques et antidépresseurs potentiels.

L'activité antidépressive a été mise en évidence par le test de potentialisation du 5-HTP chez le rat selon le protocole décrit par M. JALFRE, B. BUCHER, A. COSTON, G. MOCQUET et R.D. PORSOLT : Arch. Int. Pharmacodyn. (1982), 259, 194-221. On détermine chez le rat la dose de produit qui, administrée par voie orale, provoque chez 50 % des animaux (DE₅₀) l'apparition de tremblements généralisés ou des stéréotypies (pianotage, mouvements de tête) consécutifs à l'administration par voie intrapéritonéale 1 h après le premier traitement d'hydroxy-5 tryptophane (5-HTP). Les résultats obtenus avec certains composés selon l'invention dans le test précédent sont rassemblés, à titre d'exemple, dans le tableau ci-après

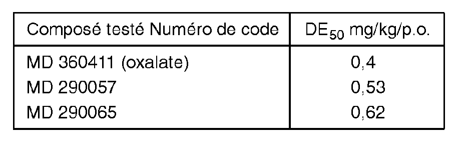

Par ailleurs, les composés selon l'invention possèdent un indice thérapeutique tel que leur emploi est sans danger aux doses actives.

Les résultats qui précèdent montrent que les composés objet de la présente invention [dérivés (1), leurs N-oxyde et sels pharmaceutiquement acceptables] peuvent être utilisés pour la préparation de médicaments psychotropes et notamment d'anxiolytiques et d'antidépresseurs potentiels, ces médicaments trouvant leur emploi en thérapeutique, notamment pour le traitement des états dépressifs endogène et exogène.

Ces médicaments peuvent être administrés à l'homme ou à tout animal à sang chaud, sous diverses formes pharmaceutiques bien connues dans la technique et notamment sous la forme de compositions formulées en vue de leur administration par voie orale, injectable ou rectale.

Pour l'administration par voie orale, lesdites compositions peuvent prendre la forme de comprimés, dragées ou gélules préparées par les techniques habituelles utilisant des supports et excipients connus tels que des agents liants, des charges, des lubrifiants et des agents de désintégration ; elles peuvent également prendre la forme de solutions, sirops ou suspensions.

Pour l'administration sous forme de soluté injectable, les compositions selon l'invention peuvent prendre la forme de solutions, suspensions ou émulsions injectables comprenant un véhicule liquide huileux ou aqueux acceptable.

Pour l'administration par voie rectale, les compositions peuvent prendre la forme de suppositoire comprenant les bases habituelles pour suppositoires.

La dose thérapeutique active des principes actifs, c'est-à-dire des dérivés (1), leurs sels pharmaceutiquement acceptables et N-oxyde, dépend notamment de la voie d'administration, du poids corporel du patient et de la puissance thérapeutique des principes actifs mis en oeuvre.

Généralement par voie orale, les doses administrées pourront atteindre 10 mg/kg/jour de principe actif (en une ou plusieurs prises) ; par voie injectable, elles pourront atteindre 1 mg/kg/jour (en une ou plusieurs prises) ; par voie rectale, elles pourront atteindre 5 mg/kg/jour de principe actif (en un ou plusieurs suppositoires.

## Revendications (Revendications pour l'(les) Etat(s) contractant(s) suivant(s) : AT, BE, CH, DE, DK, GB, IT, LI, LU, MC, NL, PT, SE)

1. Dérivés répondant à la formule : 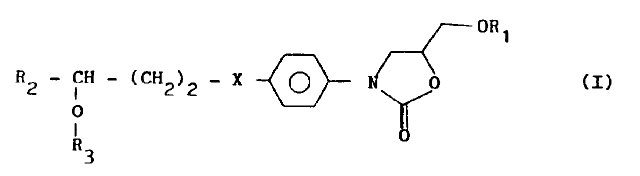 où:
- R₁ représente alkyle en C₁-C₄ ;
- X représente un atome d'oxygène ou un groupe méthylène
- R₂ représente alkyle en C₁-C₄ ou CF₃ ;
- R₃ représente un groupe CH₂R₄ dans lequel R₄ est un élément choisi dans le groupe comprenant : imidazolyle, pyrazolyle, pyridyle, pyrimidinyle, pyrazinyle, pyridazinyle, quinolyle, isoquinolyle, quinoxalinyle, quinazolinyle et cinnolinyle,
ainsi que leur éventuel N-oxyde et les sels d'addition d'acide de ces dérivés et N-oxyde, ces dérivés se présentant sous diverses formes stéréoisomères ou sous la forme d'un mélange de ces formes, y compris la forme racémique.

2. Composés selon la revendication 1, pour lesquels R₄ = imidazolyle, pyridyle, quinolyle, pyridyle N-oxyde ou quinolyle N-oxyde.

3. Composés selon la revendication 1, choisis dans le groupe comprenant :
a) ceux pour lesquels R₁ = alkyle en C₁-C₄, X représente oxygène, R2 = CF₃ et R₄ = pyridyle ;
b) ceux pour lesquels R₁ = alkyle en C₁-C₄, X représente oxygène, R2 = CF₃ et R₄ = pyridyle N-oxyde ;
c) ceux pour lesquels R₁ = alkyle en C₁-C₄, X représente oxygène, R₂ = CF₃, R₄ = pyridyle, et dont le carbone asymétrique du cycle oxazolidinone est de configuration (R) ;
d) ceux pour lesquels R₁ = alkyle en C₁-C₄, X représente oxygène, R2 = CF₃, R₄ = pyridyle N-oxyde, et dont le carbone asymétrique du cycle oxazolidinone est de configuration (R) ;
e) ceux pour lesquels R₁ = alkyle en C₁-C₄, X représente oxygène, R₂ = CF₃, R₄ = pyridyle et dont le carbone asymétrique 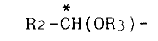 est de configuration (R) ;
f) ceux pour lesquels R₁= alkyle en C₁-C₄, X représente oxygène, R₄ = pyridyle N-oxyde et dont le carbone asymétrique 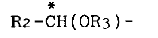 est de configuration (R) ;
g) ceux pour lesquels R₁ = alkyle en C₁-C₄, X représente oxygène, R₂ = CF₃, R₄ = pyridyle et dont le carbone asymétrique 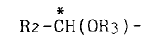 est de configuration (S) ;
h) ceux pour lesquels R₁ = alkyle en C₁-C₄, X représente oxygène. R₄ = pyridyle N-oxyde et dont le carbone asymétrique 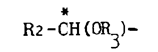 est de configuration (S) ;
i) ceux pour lesquels R₁ = alkyle en C₁-C₄, X représente oxygène, R₂ = CF₃, R₄ = pyridyle et de configuration (R, R) ;
j) ceux pour lesquels R₁ = alkyle en C₁-C4, X représente oxygène, R₂ = CF₃, R₄ = pyridyle N-oxyde et de configuration (R, R) ;
k) les dérivés selon a) à j) où X représente non pas oxygène mais un groupe CH₂ ; et
I) les dérivés selon a) à k) sous la forme de sels d'addition d'acide.

4. Composé selon la revendication 1, choisi parmi ceux de formule :

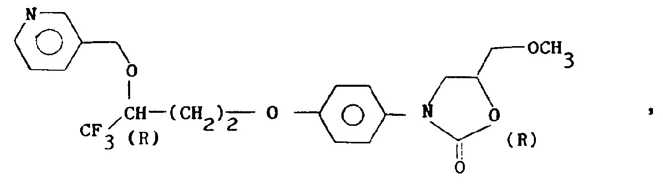

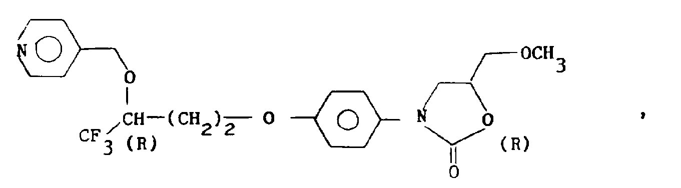

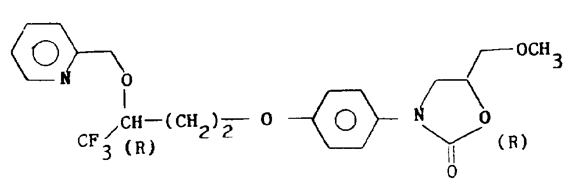

5. Composé selon la revendication 4, sous la forme N-oxyde

6. Composition pharmaceutique, caractérisée en ce qu'elle comprend un excipient physiologiquement acceptable en association avec au moins un composé selon l'une des revendications 1 à 5.

7. Utilisation des composés selon la revendication 1 à 5 pour la préparation de médicaments psychotropes.

8. Procédé de préparation des dérivés (I) selon la revendication 1, caractérisé en ce qu'il comprend l'O.alcoylation par un composé de formule 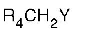 où Y = halogène et R4 a la même signification que dans la formule (1), respectivement des composés de formule: 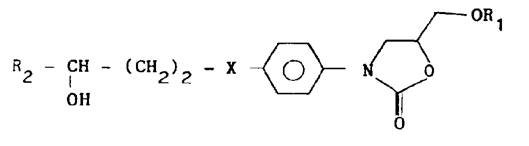 où R₁, R₂ et X ont la même signification que dans la formule (I), suivie éventuellement d'une N-oxydation et/ou salification des dérivés ainsi obtenus.

## Revendications (Revendications pour l'(les) Etat(s) contractant(s) suivant(s) : ES, GR)

1. Procédé de préparation des dérivés répondant à la formule : 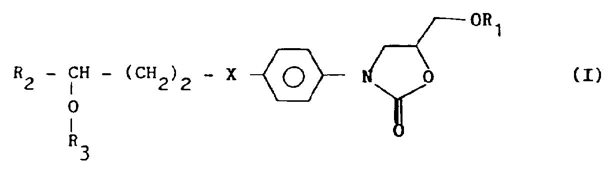 où:
- R₁ représente alkyle en C₁-C₄ ;
- X représente un atome d'oxygène ou un groupe méthylène
- R₂ représente alkyle en C₁-C₄ ou CF3 ;
- R₃ représente un groupe CH₂R₄ dans lequel R₄ est un élément choisi dans le groupe comprenant : imidazolyle, pyrazolyle, pyridyle, pyrimidinyle, pyrazinyle, pyridazinyle, quinolyle, isoquinolyle, quinoxalinyle, quinazolinyle et cinnolinyle,
ainsi que de leur éventuel N-oxyde et des sels d'addition d'acide de ces dérivés et N-oxyde, ces dérivés se présentant sous diverses formes stéréoisomères ou sous la forme d'un mélange de ces formes, y compris la forme racémique,
caractérisé en ce qu'il comprend l'O.alcoylation par un composé de formule : 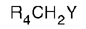 où Y = halogène et R₄ a la même signification que dans la formule (I) respectivement des composés de formule: 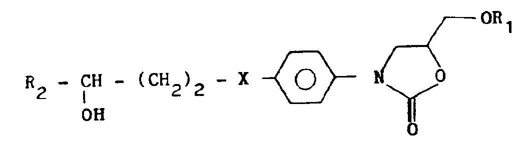 où R₁, R₂ et X ont la même signification que dans la formule (I), suivie éventuellement d'une N-oxydation et/ou salification des dérivés ainsi obtenus.

2. Procédé selon la revendication 1, dans lequel R₄ = imidazolyle, pyridyle, quinolyle, pyridyle N-oxyde ou quinolyle N-oxyde.

3. Procédé selon la revendication 1, pour la préparation de dérivés (I) choisis dans le groupe comprenant :
a) ceux pour lesquels R₁ = alkyle en C₁-C₄, X représente oxygène, R₂ = CF₃ et R₄ = pyridyle ;
b) ceux pour lesquels R₁ = alkyle en C₁-C₄, X représente oxygène, R₂ = CF₃ et R₄ = pyridyle N-oxyde ;
c) ceux pour lesquels R₁ = alkyle en C₁-C₄, X représente oxygène, R2 = CF₃, R₄ = pyridyle, et dont le carbone asymétrique du cycle oxazolidinone est de configuration (R) ;
d) ceux pour lesquels R₁ = alkyle en C₁-C₄, X représente oxygène, R2 = CF₃, R₄ = pyridyle N-oxyde, et dont le carbone asymétrique du cycle oxazolidinone est de configuration (R) ;
e) ceux pour lesquels R₁ = alkyle en C₁-C₄, X représente oxygène, R₂ = CF₃, R₄ = pyridyle et dont le carbone asymétrique 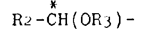 est de configuration (R) ;
f) ceux pour lesquels R₁ = alkyle en C₁-C₄, X représente oxygène, R₄ = pyridyle N-oxyde et dont le carbone asymétrique 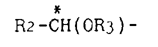 est de configuration (R) ;
g) ceux pour lesquels R₁ = alkyle en C₁-C₄, X représente oxygène, R₂ = CF₃, R₄ = pyridyle et dont le carbone asymétrique 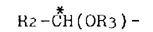 est de configuration (S);
h) ceux pour lesquels R₁ = alkyle en C₁-C₄, X représente oxygène, R₄ = pyridyle N-oxyde et dont le carbone asymétrique 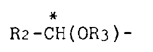 est de configuration (S) ;
i) ceux pour lesquels R₁ = alkyle en C₁-C₄, X représente oxygène, R₂ = CF₃, R₄ = pyridyle et de configuration (R, R);
j) ceux pour lesquels R₁ = alkyle en C₁-C₄, X représente oxygène, R₂ = CF₃, R₄ = pyridyle N-oxyde et de configuration (R, R) ;
k) les dérivés selon a) à j) où X représente non pas oxygène mais un groupe CH₂ ; et
I) les dérivés selon a) à k) sous la forme de sels d'addition d'acide.

4. Procédé selon la revendication 1, pour la préparation d'un dérivé choisi parmi ceux de formule : 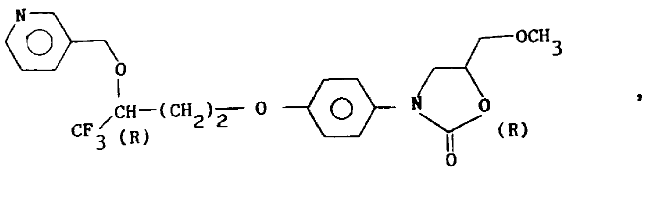 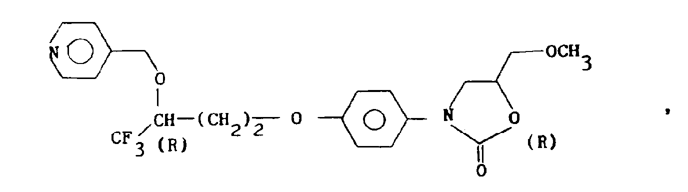 ou

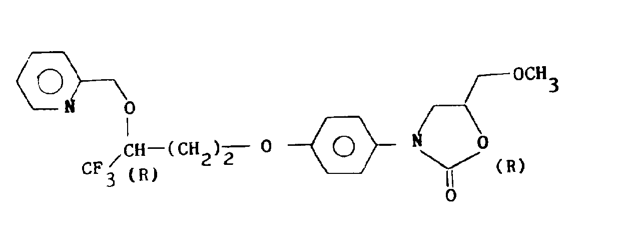

5. Procédé selon la revendication 4, dans lequel le dérivé est sous la forme N-oxyde.

6. Utilisation des dérivés répondant à la formule 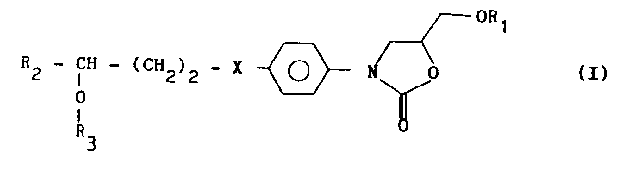 où:
- R₁ représente alkyle en C₁-C₄;
- X représente un atome d'oxygène ou un groupe méthylène
- R₂ représente alkyle en C₁-C₄ ou CF₃ ;
- R₃ représente un groupe CH₂R₄ dans lequel R₄ est un élément choisi dans le groupe comprenant : imidazolyle, pyrazolyle, pyridyle, pyrimidinyle, pyrazinyle, pyridazinyle, quinolyle, isoquinolyle, quinoxalinyle, quinazolinyle et cinnolinyle, ainsi que de leur éventuel N-oxyde et des sels d'addition d'acide de ces dérivés et N-oxyde, ces dérivés se présentant sous diverses formes stéréoisomères ou sous la forme d'un mélange de ces formes, y compris la forme racémique,
pour la préparation de médicaments psychotropes.

## Patentansprüche (Patentansprüche für folgende(n) Vertragsstaat(en) : AT, BE, CH, DE, DK, GB, IT, LI, LU, MC, NL, PT, SE)

1. Derivate gemäß der Formel: 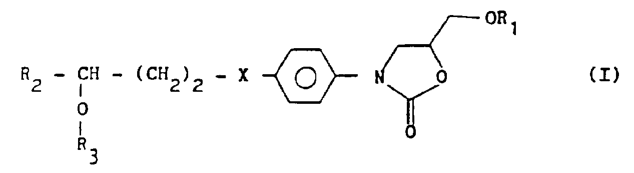 worin:
- R₁ C₁-C₄-Alkyl darstellt;
- X ein Sauerstoffatom oder eine Methylengruppe darstellt;
- R₂ C₁-C₄-Alkyl oder CF₃ darstellt;
- R₃ eine CH₂R₄-Gruppe darstellt, in welcher R₄ ein Vertreter gewählt aus der Gruppe umfassend: Imidazolyl, Pyrazolyl, Pyridyl, Pyrimidinyl, Pyrazinyl, Pyridazinyl, Chinolyl, Isochinolyl, Chinoxalinyl, Chinazolinyl und Cinnolinyl ist
sowie gegebenenfalls ihr N-Oxid und die Säureadditionssalze dieser Derivate und ihres N-Oxids, wobei diese Derivate in verschiedenen stereoisomeren Formen oder in Form eines Gemisches dieser Formen einschließlich des Racemats vorliegen.

2. Verbindungen nach Anspruch 1, worin R4 = Imidazolyl, Pyridyl, Chinolyl, Pyridyl-N-oxid oder Chinolyl-N-Oxid.

3. Verbindungen nach Anspruch 1, gewählt aus der Gruppe umfassend:
a) diejenigen, worin R₁ = C₁-C₄-Alkyl, X Sauerstoff darstellt, R₂ = CF₃ und R₄ = Pyridyl:
b) diejenigen, worin R₁ = C₁-C₄-Alkyl, X Sauerstoff darstellt, R₂ = CF₃ und R₄ = Pyridyl-N-Oxid;
c) diejenigen, worin R₁ = C₁-C₄-Alkyl, X Sauerstoff darstellt, R₂= CF3, R₄ = Pyridyl, und deren asymmetrisches Kohlenstoffatom des Oxazolidinonrings die (R)-Konfiguration hat;
d) diejenigen, worin R₁ = C₁-C₄-Alkyl, X Sauerstoff darstellt, R₂ = CF₃, R₄ = Pyridyl-N-Oxid, und deren asymmetrisches Kohlenstoffatom des Oxazolidinonrings die (R)-Konfiguration hat;
e) diejenigen, worin R₁ = C₁-C₄-Alkyl, X Sauerstoff darstellt, R2 CF₃, R₄ = Pyridyl und deren asymmetrisches Kohlenstoffatom R₂-C*H(OR₃)- die (R)-Konfiguration hat;
f) diejenigen, worin R₁ = C₁-C₄-Alkyl, X Sauerstoff darstellt, R₄ = Pyridyl-N-Oxid und deren asymmetrisches Kohlenstoffatom R₂-C*H(OR₃) - die (R)-Konfiguration hat;
g) diejenigen, worin R₁ = C₁-C₄-Alkyl, X Sauerstoff darstellt, R₂= CF3, R₄ = Pyridyl und deren asymmetrisches Kohlenstoffatom R₂-C*H(OR₃)- die (S)-Konfiguration hat;
h) diejenigen, worin R₁ = C₁-C₄-Alkyl, X Sauerstoff darstellt, R₄ = Pyridyl-N-Oxid und deren asymmetrisches Kohlenstoffatom R₂-C*H(OR₃)- die (S)-Konfiguration hat;
i) diejenigen, worin R₁ = C₁-C₄-Alkyl, X Sauerstoff darstellt, R₂ = CF₃, R₄ = Pyridyl und mit der (R,R)-Konfiguration;
j) diejenigen, worin R₁ = C₁-C₄-Alkyl, X Sauerstoff darstellt, R₂ = CF₃, R₄ = Pyridyl-N-oxid und mit der (R,R)-Konfiguration;
k) die Derivate nach a) bis j), worin X nicht Sauerstoff sondern eine CH₂-Gruppe darstellt, und
I) die Derivate nach a) bis k) in Form von Säureadditionssalzen.

4. Verbindung nach Anspruch 1, gewählt aus denjenigen mit der Formel: 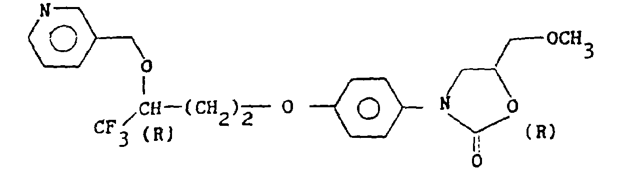 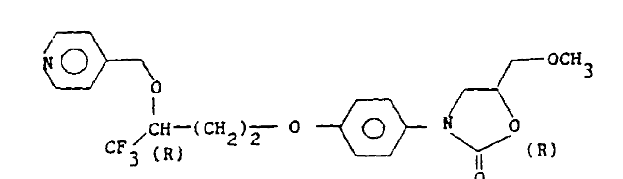 oder

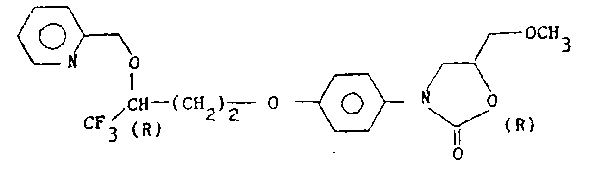

5. Verbindung nach Anspruch 4, in Form des N-Oxids.

6. Pharmazeutische Zusammensetzung, dadurch gekennzeichnet, daß sie ein physiologisch annehmbares Excipiens zusammen mit mindestens einer Verbindung nach einem der Ansprüche 1 bis 5 umfaßt.

7. Verwendung der Verbindungen nach Anspruch 1 bis 5 zur Herstellung von psychotropen Arzneimitteln.

8. Verfahren zum Herstellen von Derivaten (I) nach Anspruch 1, dadurch gekennzeichnet, daß es die O-Alkylierung durch eine Verbindung der Formel: 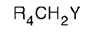 worin Y = Halogen und R₄ die gleiche Bedeutung wie in Formel (I) hat, von Verbindungen der Formel: 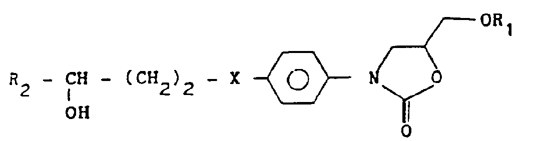 worin R₁, R₂ und X die gleiche Bedeutung wie in Formel (I) haben,
gegebenenfalls gefolgt von einer N-Oxidation und/oder Bildung von Salzen der so erhaltenen Derivate umfaßt.

## Patentansprüche (Patentansprüche für folgende(n) Vertragsstaat(en) : ES, GR)

1. Verfahren zum Herstellen von Derivaten gemäß der Formel: 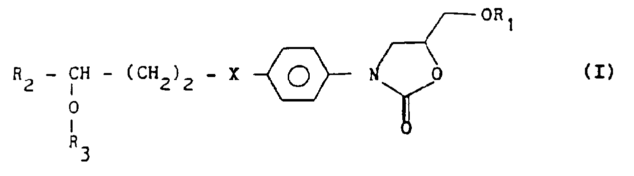 worin:
- R₁ C₁-C₄-Alkyl darstellt;
- X ein Sauerstoffatom oder eine Methylengruppe darstellt;
- R₂ C₁-C₄-Alkyl oder CF₃ darstellt;
- R₃ eine CH₂R₄-Gruppe darstellt, in welcher R₄ ein Vertreter gewählt aus der Gruppe umfassend: Imidazolyl, Pyrazolyl, Pyridyl, Pyrimidinyl, Pyrazinyl, Pyridazinyl, Chinolyl, Isochinolyl, Chinoxalinyl, Chinazolinyl und Cinnolinyl ist
sowie gegebenenfalls ihres N-Oxids und der Säureadditionssalze dieser Derivate und ihres N-Oxids, wobei diese Derivate in verschiedenen stereoisomeren Formen oder in Form eines Gemisches dieser Formen einschließlich des Racemats vorliegen, dadurch gekennzeichnet, daß es die 0-Alkylierung durch eine Verbindung der Formel:
R₄CH₂Y
worin Y = Halogen und R₄ die gleiche Bedeutung wie in Formel (I) hat, von Verbindungen der Formel: 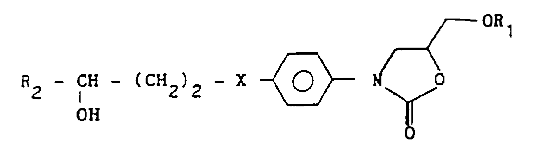 worin R₁, R₂ und X die gleiche Bedeutung wie in Formel (I) haben,
gegebenenfalls gefolgt von einer N-Oxidation und/oder Bildung von Salzen der so erhaltenen Derivate umfaßt.

2. Verfahren nach Anspruch 1, worin R₄ = Imidazolyl, Pyridyl, Chinolyl, Pyridyl-N-Oxid oder Chinolyl-N-Oxid.

3. Verfahren nach Anspruch 1, zur Herstellung von Derivaten (I) gewählt aus der Gruppe umfassend:
a) diejenigen, worin R₁ = C₁-C₄-Alkyl, X Sauerstoff darstellt, R₂ = CF₃ und R₄ = Pyridyl;
b) diejenigen, worin R₁ = C₁-C₄-Alkyl, X Sauerstoff darstellt, R₂ = CF₃ und R₄ = Pyridyl-N-Oxid;
c) diejenigen, worin R₁ = C₁-C₄-Alkyl, X Sauerstoff darstellt, R2=CF3, R₄ = Pyridyl, und deren asymmetrisches Kohlenstoffatom des Oxazolidinonrings die (R)-Konfiguration hat;
d) diejenigen, worin R₁ = C₁-C₄-Alkyl, X Sauerstoff darstellt, R₂ = CF₃, R₄ = Pyridyl-N-Oxid und deren asymmetrisches Kohlenstoffatom des Oxazolidinonrings die (R)-Konfiguration hat;
e) diejenigen, worin R₁ = C₁-C₄-Alkyl, X Sauerstoff darstellt, R₂ = CF₃, R₄ = Pyridyl und deren asymmetrisches Kohlenstoffatom R₂-C*H(OR₃)- die (R)-Konfiguration hat;
f) diejenigen, worin R₁ = C₁-C₄-Alkyl, X Sauerstoff darstellt, R₄ = Pyridyl-N-Oxid und deren asymmetrisches Kohlenstoffatom R₂-C*H(OR₃)- die (R)-Konfiguration hat;
g) diejenigen, worin R₁ = C₁-C₄-Alkyl, X Sauerstoff darstellt, R₂= CF₃, R4 = Pyridyl und deren asymmetrisches Kohlenstoffatom R₂-C*H(OR₃)- die (S)-Konfiguration hat;
h) diejenigen, worin R₁ = C₁-C₄-Alkyl, X Sauerstoff darstellt, R₄ = Pyridyl-N-Oxid und deren asymmetrisches Kohlenstoffatom R₂-C*H(OR₃)- die (S)-Konfiguration hat;
i) diejenigen, worin R₁ = C₁-C₄-Alkyl, X Sauerstoff darstellt, R₂ = CF₃, R₄ = Pyridyl und mit der (R,R)-Konfiguration;
j) diejenigen, worin R₁ = C₁-C₄-Alkyl, X Sauerstoff darstellt, R₂ = CF₃, R₄ = Pyridyl-N-Oxid und mit der (R,R)-Konfiguration;
k) die Derivate nach a) bis j), worin X nicht Sauerstoff sondern eine CH₂-Gruppe darstellt, und
I) die Derivate nach a) bis k) in Form von Säureadditionssalzen.

4. Verfahren nach Anspruch 1, zur Herstellung eines Derivats, gewählt aus denjenigen mit der Formel: 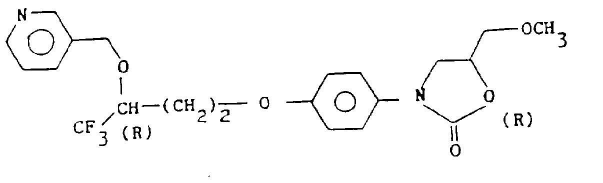 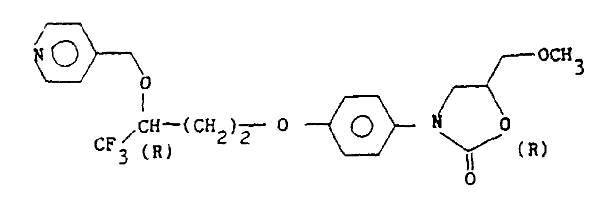 oder

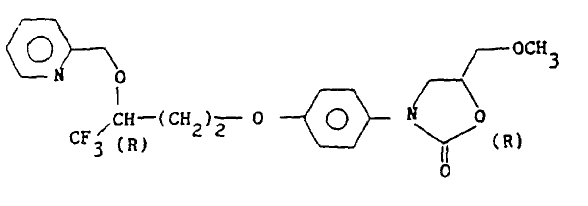

5. Verfahren nach Anspruch 4, worin das Derivat in Form des N-Oxids vorliegt.

6. Verwendung von Derivaten gemäß der Formel: 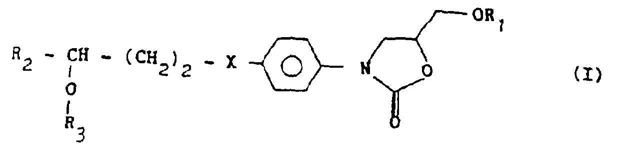 worin:
- R₁ C₁-C₄-Alkyl darstellt;
- X ein Sauerstoffatom oder eine Methylengruppe darstellt;
- R₂ C₁-C₄-Alkyl oder CF₃ darstellt;
- R₃ eine CH₂R₄-Gruppe darstellt, in welcher R₄ ein Vertreter gewählt aus der Gruppe umfassend: Imidazolyl, Pyrazolyl, Pyridyl, Pyrimidinyl, Pyrazinyl, Pyridazinyl, Chinolyl, Isochinolyl, Chinoxalinyl, Chinazolinyl und Cinnolinyl ist sowie gegebenenfalls ihres N-Oxids und der Säureadditionssalze dieser Derivate und ihres N-Oxids, wobei diese Derivate in verschiedenen stereoisomeren Formen oder in Form eines Gemisches dieser Formen einschließlich des Racemats vorliegen,
zur Herstellung von psychotropen Arzneimitteln.

## Claims (Claims for the following Contracting State(s) : AT, BE, CH, DE, DK, GB, IT, LI, LU MC, NL, PT, SE.)

1. Derivatives having the formula: 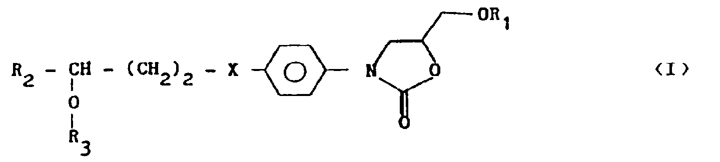 where:
- R₁ represents C₁-C₄ alkyl;
- X represents an oxygen atom or a methylene group;
- R₂ represents C₁-C₄ alkyl or CF₃;
- R₃ represents a CH₂R₄ group in which R₄ is a member chosen from the group comprising : imidazolyl, pyrazolyl, pyridyl, pyrimidinyl, pyrazinyl, pyridazinyl, quinolyl, isoquinolyl, quinoxalinyl, quinazolinyl and cinnolinyl, and their N-oxide if any and the acid addition salts of these derivates and N-oxide, the derivatives being in various stereoisomeric forms or in the form of a mixture of these forms, including the racemic form

2. Process according to claim 1, in which R₄ = imidazolyl, pyridyl, quinolyl, pyridyl N-oxide or quinolyl N-oxide.

3. Process according to claim 1, for preparing derivatives (I) chosen from the group comprising :
a) those in which R₁ = C₁-C₄ alkyl, X denotes oxygen, R₂ = CF₃ and R₄ = pyridyl;
b) those in which R₁ = C₁-C₄ alkyl, X denotes oxygen, R₂ = CF₃ and R₄ = pyridyl N-oxide;
c) those in which R₁ = C₁-C₄ alkyl, X denotes oxygen, R₂ = CF₃, R₄ = pyridyl, and the asymmetrical carbon in the oxazolidinone ring has the configuration (R);
d) those in which R₁ = C₁-C₄ alkyl, X denotes oxygen, R₂ = CF₃, R₄ = pyridyl N-oxide, and the asymmetrical carbon in the oxazolidinone ring has the configuration (R);
e) those in which R₁ = C₁-C₄ alkyl, X denotes oxygen, R₂ = CF₃, R₄ = pyridyl and the asymmetrical carbon 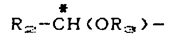 has the configuration (R);
f) those in which R₁ = C₁-C₄ alkyl, X denotes oxygens, R₄ = pyridyl N-oxide and the asymmetrical carbon 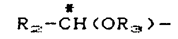 has the configuration (R);
g) those in which R₁ = C₁-C₄ alkyl, X denotes oxygen, R₂ = CF₃, R₄ = pyridyl arid the asymmetrical carbon 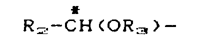 has the configuration (S);
h) those in which R₁ = C₁-C₄ alkyl, X denotes oxygen, R₄ = pyridyl N-oxide and the asymmetrical carbon 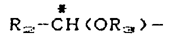 has the configuration (S);
i) those in which R₁ = C₁-C₄ alkyl, X denotes oxygen, R2, CF3, R₄ = pyridyl and the configuration is (R, R);
j) those in which R₁ = C₁-C₄ alkyl, X denotes oxygen, R₂ = CF₃, R₄ = pyridyl N-oxide and the configuration is (R, R);
k) derivatives as per a) to j) where X represents a CH₂ group instead of oxygen ; and
I) derivatives as per a) to k) in the form of acid addition salts.

4. A compound according to claim 1, chosen from among those having the formula: 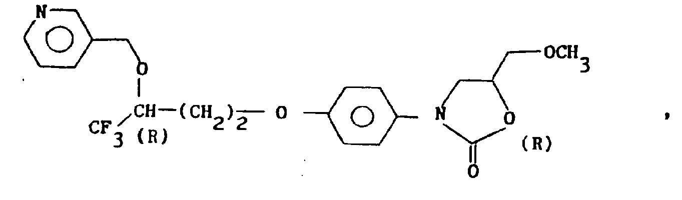 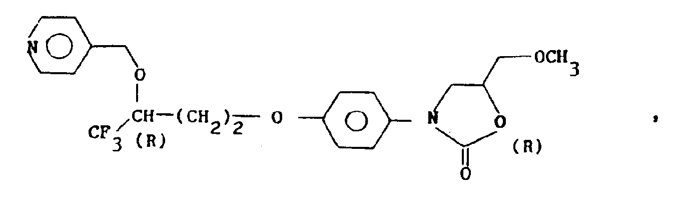 or

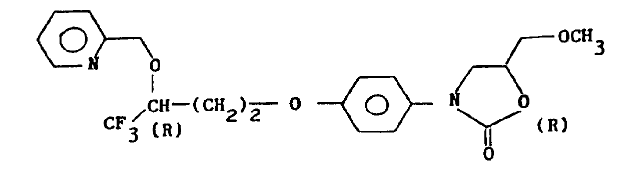

5. A compound according to claim 4, in the N-oxide form

6. A pharmaceutical composition characterised in that it contains a physiologically acceptable excipient in association with at least one compound according to any of claims 1 to 5.

7. Use of the compounds according to claims 1 to 5 for preparing psychotropic drugs.

8. A method of prepacking the derivatives (I) according to claim 1, characterised in that it comprises O-alkylation by a compound having the formula: 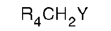 where Y = halogen and R₄, has the same meaning as in formula (I), of respective compounds having the formula: 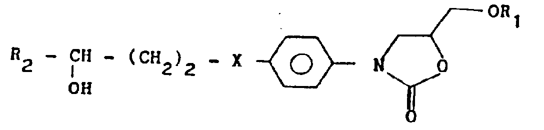 where R_{1,} R₂ and X have the same meaning as in formula (I), optionally followed by N-oxidation and/or salification or the resulting derivatives,

## Claims (Claims for the following Contracting State(s) : ES, GR)

1. Process of preparing derivatives having the formula : 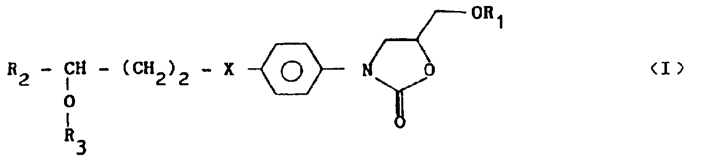 where:
- R₁ represents C₁-C₄ alkyl;
- X represents an oxygen atom or a methylene group;
- R₂ represents C₁-C₄ alkyl or CF₃;
- R₃ represents a CH₂R₄, group in which R₄ is a member chosen from the group comprising : imidazolyl, pyrazolyl, pyridyl, pyrimidinyl, pyrazinyl, pyridazinyl, quinolyl, isoquinolyl, quinoxalinyl, quinazolinyl and cinnolinyl, and of their N-oxide if any and of the acid addition salts of these derivatives and N-oxide, the derivatives being in various stereoisomeric forms or in the form of a mixture of these forms, including the racemic form, characterized in that it comprises O-alkylation by a compound having the formula : 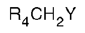 where Y = halogen and R₄ has the same meaning as in formula (I), of respective compounds having the formula: 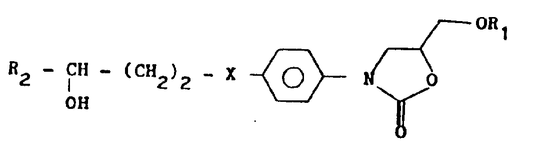 where R₁, R₂ and X have the same meaning as in formula (I), optionally followed by N-oxidation and/or salification of the resulting derivatives.

2. Process accordions to claim 1, in which R₄ = imidazolyl, pyridyl, quinolyl, pyridyl N-oxide or quinolyl N-oxide.

3. Process according to claim 1, for preparing derivatives (I) chosen from the group comprising :
a) those in which R₁ = C₁-C₄ alkyl, X denotes oxygen, R₂ = CF₃ and R₄ = pyridyl;
b) those in which R₁ = C₁-C₄ alkyl, X denotes oxygen, R₂ = CF₃, and R₄ = pyridyl N-oxide;
c) those in which R₁ = C₁-C₄ alkyl, X denotes oxygen, R₂ = CF₃, R₄ = pyridyl, and the asymmetrical carbon in the oxazolidinone ring has the configuration (R);
d) those in which R₁ = C₁-C₄ alkyl, X denotes oxygen, R₂ = CF₃, R₄ = pyridyl N-oxide, and the asymmetrical carbon in the oxazolidinoiie ring has the configuration (R);
e) those in which R₁ = C₁-C₄, alkyl, X denotes oxygen, R₂ = CF₃, R₄ = pyridyl and the asymmetrical carbon 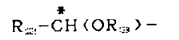 has the configuration (R);
f) those in which R₁ = C₁-C₄ alkyl, X denotes oxygen, R₄ = pyridyl N-oxide and the asymmmetrical carbon 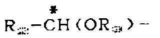 has the configuration (R);
g) those in which R₁ = C₁-C₄ alkyl, X denotes oxygen, R₂ CF3, R₄, = pyridyl and the asymmetrical carbon 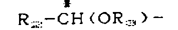 has the configuration (S);
h) those in which R₁ = C₁-C₄ alkyl, X denotes oxygen, R₄ = pyridyl N-oxide and the asymmetrical carbon 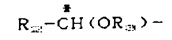 has the configuration (S);
i) those in which R₁ = C₁-C₄ alkyl, X denotes oxygen, R₂ = CF₃, R₄, = pyridyl and the configuration is (R, R);
j) those in which R₁ = C₁-C₄ alkyl, X denotes oxygen, R₂ = CF₃, R4 = pyridyl N-oxide and the configuration is (R, R);
k) derivatives as per a) to j) where X represents a CH₂ group instead of oxygen ; and
I) derivatives as per a) to k) in the form of acid addition salts.

4. Process according to claim 1, for preparing a derivative chosen from among those having the formula: 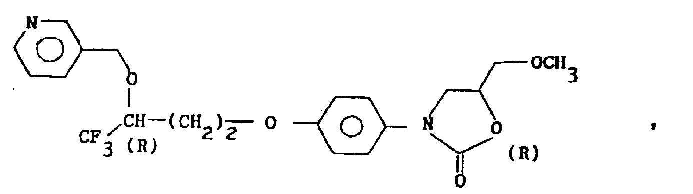 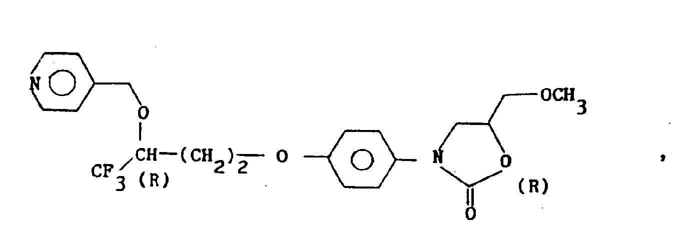 or

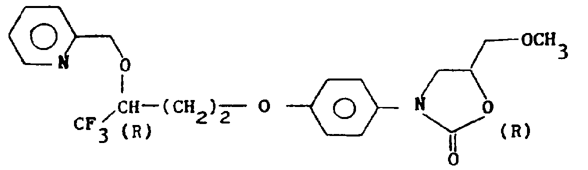

5. Process according to claim 4, wherein the derivative is in the N-oxide form.

6. Use of the derivatives having the formula : 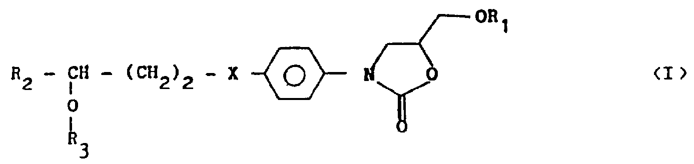 where:
R₁ represents C₁-C₄ alkyl;
X represents an oxygen atom or a methylene group;
R₂ represents C₁-C₄ alkyl or CF₃;
R₃ represents a CH₂R₄ group in which R₄ is a member chosen from the group comprising : imidazolyl, pyrazolyl, pyridyl, pyrimidinyl, pyrazinyl, pyridazinyl, quinolyl, isoquinolyl, quinoxalinyl, quinazolinyl and cinnolinyl,
and of their N-oxide if any and of the acid addition salts of these derivatives and N-oxide, the derivatives being in various stereoisomeric forms or in the form of a mixture of these forms, including the racemic form, for the preparation of psychotropic drugs.
